# EUROPEAN PATENT APPLICATION

(11) **EP 4 847 024 A2**
(43) Date of publication of application: **02.12.2026**
(21) Application number: 26210023.3
(22) Date of filing: 18.12.2024
(51) Int. Cl.: A61K 31/549

(54) **IMMEDIATE RELEASE MONOLAYER TABLET COMPRISING TELMISARTAN AND ONE OR MORE OTHER ANTIHYPERTENSIVE AGENTS, METHOD OF MANUFACTURING THE SAME, AND ITS USE**

(30) Priority: 20.12.2023 EP 23218918; 25.04.2024 EP 24172505
(62) Document number(s) of the earlier application(s) in accordance with Art. 76 EPC: 24833965.7 / 4 827 803
(71) Applicant: KRKA, d.d., Novo mesto, 8501 Novo mesto (SI)
(72) Inventor: KOSMAC, Ina, 8000 Novo Mesto (SI); MIRTIC, Janja, 8000 Novo Mesto (SI); HRVATIC, Nina, 8000 Novo Mesto (SI); MAKSE, Matej, 8000 Novo Mesto (SI); RITLOP, Gregor, 8000 Novo Mesto (SI); HROVAT, Irena, 8000 Novo Mesto (SI); HUDOVORNIK, Grega, 8000 Novo Mesto (SI)
(74) Representative: Ter Meer und Partner mbB

(57) **Abstract**

The present invention relates to a monolayer tablet for immediate release, a method of manufacturing the monolayer tablet, and the use of the monolayer tablet in treating essential hypertension or/and reducing the risk of development of a condition associated with essential hypertension. The monolayer tablet comprises (a) an intragranular phase comprising (al) amorphous telmisartan and (a2) alkaline agent; and (b) an extragranular phase comprising (bl) one or more other antihypertensive agents selected from amlodipine, hydrochlorothiazide and indapamide, and (b2) disintegrant.

## Description

### Field of the invention

The present invention relates to a monolayer tablet for immediate release, a method of manufacturing the monolayer tablet, and the use of the monolayer tablet in treating essential hypertension or/and reducing the risk of development of a condition associated with essential hypertension. In particular, the present invention relates to a monolayer tablet comprising telmisartan and one or more other hypertensive agents selected from amlodipine, hydrochlorothiazide and indapamide.

### Background art

Hypertension, also commonly known as high blood pressure, is a medical condition in which the arterial blood pressure is persistently elevated and which represents a major cause of premature death worldwide. According to an estimation provided on the website of the World Health Organization (WHO) in early 2023, about 1.28 billion adults aged 30-79 years worldwide suffer from hypertension, with two-thirds of them living in low and middle-income countries and only about 46% being aware of their medical condition.

Risk factors for the development of hypertension include both modifiable risk factors, such as physical inactivity, unhealthy diets, consumption of tobacco and alcohol, and non-modifiable risk factors, such as a family history of hypertension and co-existing diseases such as diabetes or kidney disease. Despite the existence of effective and affordable techniques, hypertension is not always well detected, especially since it is usually not accompanied by any symptoms. Only in case of very high blood pressures can symptoms, such as severe headaches, chest pain, dizziness, nausea, blurred vision, anxiety and abnormal heart rhythm, be experienced.

Although the manipulation of modifiable risk factors can help to lower the blood pressure, most subjects suffering from hypertension require antihypertensive drug treatment alongside lifestyle modifications. In the 2023 hypertension guidelines of the European Society of Hypertension (ESH), five major drug classes are recommended as first-line agents for antihypertensive drug treatment, namely angiotensin-converting-enzyme inhibitors, angiotensin receptor blockers, calcium channel blockers, thiazide/thiazide-like diuretics and beta blockers. These drugs can be used alone or in combination, with initiation of therapy with a single drug being reserved for rare, specifically defined situations.

Typically, therapy is initiated with a two-drug combination and switched to a three-drug combination if, upon administration of the maximum recommended and tolerated dose of both drugs, the blood pressure of the hypertensive subject cannot be adequately controlled with the initial two-drug combination. Preferred two-drug combinations comprise a renin-angiotensin system (RAS) blocker combined with a calcium channel blocker or a thiazide/thiazide-like diuretic (Mancia et al., "2023 ESH Guidelines for the management of arterial hypertension", J. Hypertension 41(12) (2023), 1874-2071). The RAS blocker may be an angiotensin-converting-enzyme inhibitor or an angiotensin receptor blocker.

Specific examples of angiotensin receptor blockers (ARBs) suitable for use in a two-drug or three-drug combination comprise azilsartan, candesartan, eprosartan, irbesartan, losartan, olmesartan, telmisartan and valsartan. As specific examples of calcium channel blockers (CCBs), benzothiazepines such as diltiazem, dihydropyridines such as amlodipine, felodipine, isradipine, nifedipine, nilvadipine and nitrendipine, and phenylalkylamines such as gallopamil and verapamil are adopted. Thiazide/thiazide-like diuretics suitable for use in an antihypertensive composition comprise, among others, chlortalidone, chlorthiazide, clopamide, hydrochlorothiazide, indapamide and xipamide.

Considering the plurality of possible combinations conferred by the aforementioned lists of compounds, the provision of an efficient and simultaneously safe antihypertensive therapy employing an angiotensin receptor blocker in combination with a calcium channel blocker or/and a thiazide/thiazide-like diuretic is a demanding task. In addition, it has turned out that adherence of hypertensive patients to a specific antihypertensive therapy generally decreases if the antihypertensive therapy requires a treatment with two or more drugs which are to be taken separately as combination therapy. By this means, adequate control of blood pressure cannot always be achieved.

Single-pill combination (SPC) therapy, by reducing the number of pills to be taken by a hypertensive patient, is known to lead to improved adherence and persistence compared with free-equivalent combination (FEC) therapy, and thereby achieves better blood pressure control (Parati et al., "Adherence to single-pill versus free-equivalent combination therapy in hypertension", Hypertension 77 (2021), 692-705). In order to overcome the deficiencies encountered with the separate administration of drugs, manufacturers of pharmaceutical products have tried to provide single dosage forms having more than one antihypertensive agent contained therein.

In the art fixed-drug combinations of telmisartan with other antihypertensive agents, e.g. amlodipine, have been regarded as difficult. EP 1 814 527 B1 points out that standard formulation approaches of directly mixing the active components with the necessary excipients cannot be applied to a fixed dose combination of telmisartan and amlodipine and more sophisticated techniques are needed to separate the basic telmisartan formulation from the amlodipine drug substance. Under these circumstances, only special techniques like perlonget coating, or bilayer tablet technology could be used.

Accordingly, EP 1 814 527 B1 discloses a bilayer tablet comprising telmisartan as the angiotensin receptor blocker and amlodipine as a calcium channel blocker. The bilayer tablet comprises (a) a first layer of telmisartan in a dissolving tablet matrix comprising a basic agent selected from alkali metal hydroxides, basic amino acids and meglumine, a water-soluble diluent and optionally other excipients, and (b) a second layer of amlodipine in a disintegrating or eroding tablet matrix. The dissolving tablet matrix of the first layer and the disintegrating or eroding tablet matrix of the second layer in each case designate a tablet base formulation having instant release characteristics.

CN 101 450 211 A, on the other hand, tries to overcome the prejudices that the formulation of telmisartan and amlodipine requires bi-layer technology. Accordingly, the citation tries to provide a pharmaceutical formulation prepared by direct compression, having three antihypertensive agents combined, namely an angiotensin receptor blocker selected from candesartan, eprosartan, irbesartan, losartan, olmesartan, telmisartan and valsartan, a calcium channel blocker selected from amlodipine, felodipine, lacidipine, nicardipine, nifedipine and nitrendipine, and a thiazide/thiazide-like diuretic selected from hydrochlorothiazide and indapamide.

WO 2011/025467 A1 teaches a monolayer tablet comprising telmisartan as the angiotensin receptor blocker and hydrochlorothiazide as a thiazide/thiazide-like diuretic. The monolayer tablet is obtained by granulating diluents with a granulation solution comprising telmisartan, an alkaline agent and a buffering agent so as to obtain telmisartan-containing granules, mixing the telmisartan-containing granules with a dry-blend containing hydrochlorothiazide so as to obtain a mixture, and compressing the mixture so as to obtain a monolayer tablet comprising the antihypertensive agents together with 1 to 20% by weight of buffering agent.

WO 2018/030559 A1 suggests a monolayer tablet comprising telmisartan in combination with either amlodipine as a calcium channel blocker or hydrochlorothiazide as a thiazide/thiazide-like diuretic. For this purpose, telmisartan is granulated together with binder, disintegrant, glidant and other excipients including an aluminum magnesium silicate compound and anhydrous calcium hydrogen phosphate so as to obtain telmisartan-containing granules having the telmisartan adsorbed onto the aluminum magnesium silicate compound, the telmisartan-containing granules are mixed with an amlodipine- or hydrochlorothiazide-containing formulation so as to obtain a blend, and the blend is compressed.

### Summary of the invention

### Problem to be solved

According to EP 1 814 527 B1, the bilayer tablet disclosed therein combines adequate drug stability, acceptable drug release and reliable manufacture. More specifically, the bilayer tablet is alleged to provide a largely pH-independent dissolution of poorly water-soluble telmisartan, thereby facilitating dissolution of the drug at a physiological pH level, as well as adequate stability and drug release of amlodipine. In particular, it is emphasized that the bilayer structure overcomes the stability problem caused by the incompatibility of amlodipine with the alkaline environment of the telmisartan formulation, which alkaline environment is required for increasing solubility of telmisartan in aqueous systems.

However, the production of bilayer tablets is associated with certain drawbacks. In particular, a bilayer tablet is a heterogeneous system which is composed of two different layers that are separated by an interface within its final single body, and in which control of the release kinetics is primarily achieved by the composition of each layer. The properties of the finished tablet depend on the quantitative and qualitative composition, but also on the tendency to (interlayer and intralayer) deformation during the various production stages. Hence, the manufacture of bilayer tablets requires a precise choice of excipients and conditions with regard to each production step (T. Blicharski et al., "Challenges in technology of bilayer and multilayer tablets: a mini-review", Curr. Issues Pharm. Med. Sci. 32(4) (2019), 229-235).

Specific challenges during development of bilayer tablets might include inadequate hardness, the order of layer sequence, layer weight ratio, and elastic mismatch of the adjacent layers, first layer tamping force and cross contamination between layers. If these elements are not adequately controlled, they will have an adverse effect on bilayer compression pressure, as well as qualitative characteristics such as mechanical strength and individual layer weight control (A. Singh et al., "The challenges of producing bilayer tablet: A review", J. Drug Deliv. Ther., 11(4-S) (2021), 171-175). In summary, the production of bilayer tablets is both technically demanding and cost intensive.

In order to overcome the problems encountered with bilayer tablets and to ensure easy and cheap manufacture of an oral dosage form comprising a plurality of antihypertensive agents, CN 101 450 211 A proposes to combine telmisartan and amlodipine (together with indapamide) in a single layer. However, the manufacture of these monolayer tablets is associated with undesirable difficulties. For example, the described methods might face the problem that the blend to be compressed might stick to the die of the tablet press, or/and that, due to variations in the density of the feed material and partial or incomplete filling of the die, weight variations might occur.

Moreover, there might be problems arising from the specific combination of active pharmaceutical ingredients (APIs) as such. In particular, since the monolayer tablet of CN 101 450 211 A does not provide any alkaline environment, telmisartan has a poor dissolution profile and is only moderately released from the monolayer tablet upon contact with an aqueous system. In particular, it turned out that a monolayer tablet obtained by direct compression and comprising each of telmisartan, amlodipine and indapamide as antihypertensive agents provides a dissolution profile of telmisartan which is far inferior to that achieved by the bilayer tablet of EP 1 814 527 B1.

In view of the foregoing, there is an ongoing need for an antihypertensive product that is formulated for oral delivery and that provides an improvement over the antihypertensive products of the prior art. In particular, it would be desirable to provide an orally administrable antihypertensive product that allows to effectively and safely lower the blood pressure of a hypertensive patient while simultaneously providing excellent patient compliance, that can be easily and cost-effectively manufactured and that ensures excellent stability and quick release of the active pharmaceutical ingredients upon administration to the hypertensive patient.

### Means for solving the problem

In order to solve the aforementioned problem, the present inventors carried out intense studies and recognized that an orally administrable antihypertensive product allowing to effectively and safely lower the blood pressure of a hypertensive patient while simultaneously providing excellent patient compliance can be reliably obtained by combining telmisartan with at least one other antihypertensive agent selected from amlodipine, hydrochlorothiazide and indapamide and formulating the antihypertensive product as a monolayer tablet. Moreover, by formulating the antihypertensive product as a monolayer tablet, production of the product can be simplified and carried out at low cost.

Moreover, it was surprisingly found that, by formulating the monolayer tablet for immediate release and making the monolayer tablet to comprise an intragranular phase comprising amorphous telmisartan and an extragranular phase comprising the at least one other antihypertensive agent, it is not only possible to ensure a release of the active pharmaceutical ingredients which is remarkably faster than that achieved by the monolayer tablet of CN 101 450 211 A, but it is further possible to combine telmisartan with the at least one other antihypertensive agent in a single layer without affecting stability of the other antihypertensive agent(s) even if the telmisartan is contained in an alkaline environment.

By overcoming the prejudice of EP 1 814 527 B1 and the deficiencies of CN 101 450 211 A, the monolayer tablet described herein is highly suitable for antihypertensive therapy. The present invention is defined by the claims.

In a first aspect, the present invention thus relates to a monolayer tablet for immediate release. The monolayer tablet comprises
(a) an intragranular phase comprising (a1) amorphous telmisartan and (a2) alkaline agent, and
(b) an extragranular phase comprising (b1) one or more other antihypertensive agents selected from amlodipine, hydrochlorothiazide and indapamide, and (b2) disintegrant.

In a particularly preferred embodiment, the monolayer tablet of the first aspect comprises (b) an extragranular phase comprising (b1) a combination of amlodipine and indapamide as other antihypertensive agents, and (b2) disintegrant.

In a second aspect, the present invention relates to a method of manufacturing a monolayer tablet for immediate release. The method comprises the steps of:
(i) wet-granulating telmisartan in the presence of an alkaline agent, a filler and optionally further excipients so as to obtain telmisartan-containing granules;
(ii) mixing the telmisartan-containing granules with at least one antihypertensive agent selected from amlodipine, hydrochlorothiazide and indapamide, a disintegrant and optionally further excipients so as to obtain a blend;
(iii) compressing the blend so as to obtain an immediate release monolayer tablet; and
(iv) optionally coating the tablet.

In a particularly preferred embodiment, the manufacturing method of the second aspect comprises a step (ii) of mixing the telmisartan-containing granules with amlodipine, indapamide, a disintegrant and optionally further excipients so as to obtain a blend.

In a third aspect, the present invention relates to a monolayer tablet for immediate release which is obtainable by the method of the second aspect.

In a fourth aspect, the present invention relates to a monolayer tablet of the first or third aspect for use in treating essential hypertension or/and reducing the risk of development of a condition associated with essential hypertension. The monolayer tablet comprises:
(α) telmisartan at a dose of 40 mg and amlodipine at a dose of 5 mg;
(β) telmisartan at a dose of 40 mg and amlodipine at a dose of 10 mg;
(γ) telmisartan at a dose of 80 mg and amlodipine at a dose of 5 mg;
(δ) telmisartan at a dose of 80 mg and amlodipine at a dose of 10 mg;
(ε) telmisartan at a dose of 40 mg and hydrochlorothiazide at a dose of 12.5 mg;
(ζ) telmisartan at a dose of 40 mg and hydrochlorothiazide at a dose of 25 mg;
(η) telmisartan at a dose of 80 mg and hydrochlorothiazide at a dose of 12.5 mg;
(θ) telmisartan at a dose of 80 mg and hydrochlorothiazide at a dose of 25 mg;
(ι) telmisartan at a dose of 40 mg and indapamide at a dose of 2.5 mg;
(κ) telmisartan at a dose of 80 mg and indapamide at a dose of 2.5 mg;
(λ) telmisartan at a dose of 40 mg, amlodipine at a dose of 5 mg and hydrochlorothiazide at a dose of 12.5 mg;
(µ) telmisartan at a dose of 40 mg, amlodipine at a dose of 5 mg and hydrochlorothiazide at a dose of 25 mg;
(ν) telmisartan at a dose of 40 mg, amlodipine at a dose of 10 mg and hydrochlorothiazide at a dose of 12.5 mg;
(ξ) telmisartan at a dose of 40 mg, amlodipine at a dose of 10 mg and hydrochlorothiazide at a dose of 25 mg;
(ο) telmisartan at a dose of 80 mg, amlodipine at a dose of 5 mg and hydrochlorothiazide at a dose of 12.5 mg;
(π) telmisartan at a dose of 80 mg, amlodipine at a dose of 5 mg and hydrochlorothiazide at a dose of 25 mg;
(ρ) telmisartan at a dose of 80 mg, amlodipine at a dose of 10 mg and hydrochlorothiazide at a dose of 12.5 mg;
(ς) telmisartan at a dose of 80 mg, amlodipine at a dose of 10 mg and hydrochlorothiazide at a dose of 25 mg;
(σ) telmisartan at a dose of 40 mg, amlodipine a dose of 5 mg and indapamide at a dose of 2.5 mg;
(τ) telmisartan at a dose of 80 mg, amlodipine at a dose of 5 mg and indapamide at a dose of 2.5 mg; or
(υ) telmisartan at a dose of 80 mg, amlodipine at a dose of 10 mg and indapamide at a dose of 2.5 mg.

In a particularly preferred embodiment, the monolayer tablet of the fourth aspect comprises (σ) telmisartan at a dose of 40 mg, amlodipine a dose of 5 mg and indapamide at a dose of 2.5 mg; (τ) telmisartan at a dose of 80 mg, amlodipine at a dose of 5 mg and indapamide at a dose of 2.5 mg; or (υ) telmisartan at a dose of 80 mg, amlodipine at a dose of 10 mg and indapamide at a dose of 2.5 mg.

### Brief description of the drawings

Fig. 1 provides two images obtained by Raman microscopy showing the intragranular phase and the extragranular phase of a monolayer tablet for immediate release in accordance with one embodiment of the present invention (laser 532 nm, 20 × objective, analyzed surface 1500 ×1500 nm).
Fig. 2 shows the flowability of a blend of antihypertensive agents manufactured in accordance with the method of the invention (Fig. 2a), and the flowability of a blend obtained by directly blending the antihypertensive agents (Fig. 2b).
Fig. 3 is a diagram showing the dissolution profile of telmisartan when subjecting a number of differently manufactured tablets to 0.01 M hydrochloric acid.
Fig. 4 is a diagram showing the dissolution profile of amlodipine when subjecting a number of differently manufactured tablets to 0.01 M hydrochloric acid.
Fig. 5 is a diagram showing the dissolution profile of indapamide when subjecting a number of differently manufactured tablets to 0.01 M hydrochloric acid.

### Detailed description of the invention

In a first broad aspect, the invention relates to a monolayer tablet for immediate release. The monolayer tablet comprises a total of at least two active pharmaceutical ingredients (APIs) representing different drug classes of antihypertensive agents, namely an angiotensin receptor blocker, and at least one selected from a calcium channel blocker and a thiazide/thiazide-like diuretic. The monolayer tablet is formulated for immediate release, so as to ensure immediate release of all APIs upon administration to a patient and allowing quick absorption thereof in the body.

More specifically, the monolayer tablet comprises telmisartan (IUPAC name: 2-(4-{[4-methyl-6-(1-methyl-1H-1,3-benzodiazol-2-yl)-2-propyl-1H-1,3-benzodiazol-1-yl]methyl}phenyl)benzoic acid) and one or more other antihypertensive agents selected from amlodipine (IUPAC name: (*RS*)-3-ethyl 5-methyl 2-[(2-aminoethoxy)methyl]-4-(2-chlorophenyl)-6-methyl-1,4-dihydropyridine-3,5-dicarboxylate), hydrochlorothiazide (IUPAC name: 6-chloro-1,1-dioxo-3,4-dihydro-2*H*-1,2,4-benzothiadiazine-7-sulfonamide) and indapamide (IUPAC name: 4-chloro-N-(2-methyl-2,3-dihydroindol-1-yl)-3-sulfamoylbenzamide).

In the monolayer tablet described herein, each of the telmisartan, the amlodipine, the hydrochlorothiazide and the indapamide may be present in the form of the API as such (i.e. in the form of the free acid/base), in the form of a pharmaceutically acceptable salt of the API, in the form of a pharmaceutically acceptable solvate (especially a hydrate) of the API, or in the form of a pharmaceutically acceptable salt solvate (especially a salt hydrate) of the API. According to the invention, each of the APIs may be either crystalline or amorphous, the term "crystalline" being intended to encompass all possible polymorphs of a given API.

The terms "pharmaceutically acceptable salt", "pharmaceutically acceptable solvate" and "pharmaceutically acceptable salt solvate" as used herein in connection with the APIs comprised by the monolayer tablet of the invention designate a salt, a solvate or a salt solvate that has been approved or is approvable by a regulatory authority such as the European Medicines Agency (EMA) or the U.S. Food and Drug Administration (FDA), or/and that is listed in a generally recognized pharmacopoeia such as the European Pharmacopoeia or the U.S. Pharmacopoeia and has been found to be acceptable for medical use in humans or animals.

Specific examples of pharmaceutically acceptable salts are mentioned in "Remington: The Science and Practice of Pharmacy" (23th Edition, edited by A.R. Gennaro, Lippincott Williams & Wilkins, Baltimore and Philadelphia, 2020) and comprise, among others, acetate, besylate, bromide, chloride, citrate, fumarate, glucuronate, hydrobromide, hydrochloride, maleate, mesylate, nitrate, phosphate, potassium, sodium, succinate, sulfate, tartrate and tosylate. Specific examples of pharmaceutically acceptable solvates and salt solvates are known to a person skilled in the art and particularly include hydrates, which hydrates may contain either stoichiometric or non-stoichiometric amounts of water.

According to the invention, the monolayer tablet comprises (a) an intragranular phase and (b) an extragranular phase. The intragranular phase obligatorily comprises substantially amorphous telmisartan as component (a1), the term "substantially amorphous" as used herein in connection with an API meaning that at least 90%, in particular at least 95% by weight of the API is present in amorphous form, as measured by X-ray powder diffraction. Methods for manufacturing substantially amorphous telmisartan are known in the art and comprise, for example, those described in WO 03/059327 A1 and EP 1 814 527 B1.

The content of substantially amorphous telmisartan in the monolayer tablet is typically 1 to 30% by weight, preferably 2 to 25% by weight, and more preferably 5 to 20% by weight, based on the total weight of the monolayer tablet. In a preferred embodiment, telmisartan is solely contained in the intragranular phase, which means that the API is not additionally present in the extragranular phase. In the latter case, the content of telmisartan in the intragranular phase is typically 5 to 45% by weight, preferably 15 to 40% by weight, and more preferably 25 to 35% by weight, based on the total weight of the intragranular phase.

The intragranular phase further comprises alkaline agent as component (a2). The at least one alkaline agent aims at increased solubility of telmisartan in an aqueous system by deprotonating its carboxyl group. The alkaline agent can be suitably selected by a person skilled in the art and comprises, for example, alkaline metal hydroxides, such as sodium hydroxide and potassium hydroxide, alkaline earth metal hydroxides, such as magnesium hydroxide and calcium hydroxide, basic amino acids, such as arginine and lysine, basic sugar alcohols, such as meglumine, and combinations thereof.

In a preferred embodiment, the alkaline agent comprises an alkaline metal hydroxide, a basic amino acid or/and a basic sugar alcohol. More preferably, the intragranular phase comprises a combination of an alkaline metal hydroxide and a basic sugar alcohol, with a combination of sodium hydroxide and meglumine being particularly preferred.

The total content of alkaline agent in the monolayer tablet is typically 0.2 to 20% by weight, preferably 1 to 15% by weight, and more preferably 2 to 10% by weight, based on the total weight of the monolayer tablet. In a preferred embodiment, alkaline agent is solely contained in the intragranular phase, which means that the one or more alkaline agents are not additionally present in the extragranular phase. In the latter case, the content of alkaline agent in the intragranular phase is typically 0.2 to 20% by weight, preferably 1 to 15% by weight, and more preferably 2 to 10% by weight, based on the total weight of the monolayer tablet.

The extragranular phase of the monolayer tablet necessarily comprises one or more other antihypertensive agents selected from amlodipine, hydrochlorothiazide and indapamide as component (b1). Hence, the extragranular phase may comprise a single antihypertensive agent selected from amlodipine, hydrochlorothiazide and indapamide, a two-drug combination of amlodipine, hydrochlorothiazide and indapamide, or a three-drug combination of amlodipine, hydrochlorothiazide and indapamide. In any of the aforementioned cases, the amlodipine, the hydrochlorothiazide and the indapamide, independently of each other, are either crystalline or amorphous, preferably crystalline.

In a preferred embodiment, the monolayer tablet of the invention thus comprises an intragranular phase and an extragranular phase, wherein the intragranular phase comprises (a1) substantially amorphous telmisartan and (a2) alkaline agent, and the extragranular phase comprises (b1) amlodipine and (b2) disintegrant. In this embodiment, the extragranular phase does not comprise hydrochlorothiazide and indapamide, and preferably comprises amlodipine as the sole antihypertensive agent. The amlodipine is preferably crystalline.

In another preferred embodiment, the monolayer tablet of the invention comprises an intragranular phase and an extragranular phase, wherein the intragranular phase comprises (a1) substantially amorphous telmisartan and (a2) alkaline agent, and the extragranular phase comprises (b1) hydrochlorothiazide and (b2) disintegrant. In this embodiment, the extragranular phase does not comprise amlodipine and indapamide, and preferably comprises hydrochlorothiazide as the sole antihypertensive agent. The hydrochlorothiazide is preferably crystalline.

In another preferred embodiment, the monolayer tablet of the invention comprises an intragranular phase and an extragranular phase, wherein the intragranular phase comprises (a1) substantially amorphous telmisartan and (a2) alkaline agent, and the extragranular phase comprises (b1) indapamide and (b2) disintegrant. In this embodiment, the extragranular phase does not comprise amlodipine and hydrochlorothiazide, and preferably comprises indapamide as the sole antihypertensive agent. The indapamide is preferably crystalline.

In another preferred embodiment, the monolayer tablet of the invention comprises an intragranular phase and an extragranular phase, wherein the intragranular phase comprises (a1) substantially amorphous telmisartan and (a2) alkaline agent, and the extragranular phase comprises (b1) a combination of amlodipine and hydrochlorothiazide and (b2) disintegrant. In this embodiment, the extragranular phase does not comprise indapamide, and preferably comprises amlodipine and hydrochlorothiazide as the sole antihypertensive agents. The amlodipine and the hydrochlorothiazide are preferably both crystalline.

In another preferred embodiment, the monolayer tablet of the invention comprises an intragranular phase and an extragranular phase, wherein the intragranular phase comprises (a1) substantially amorphous telmisartan and (a2) alkaline agent, and the extragranular phase comprises (b1) a combination of amlodipine and indapamide and (b2) disintegrant. In this embodiment, the extragranular phase does not comprise hydrochlorothiazide, and preferably comprises amlodipine and indapamide as the sole antihypertensive agents. The amlodipine and the indapamide are preferably both crystalline.

In another preferred embodiment, the monolayer tablet of the invention comprises an intragranular phase and an extragranular phase, wherein the intragranular phase comprises (a1) substantially amorphous telmisartan and (a2) alkaline agent, and the extragranular phase comprises (b1) a combination of hydrochlorothiazide and indapamide and (b2) disintegrant. In this embodiment, the extragranular phase does not comprise amlodipine, and preferably comprises hydrochlorothiazide and indapamide as the sole antihypertensive agents. The hydrochlorothiazide and the indapamide are preferably both crystalline.

In another preferred embodiment, the monolayer tablet of the invention comprises an intragranular phase and an extragranular phase, wherein the intragranular phase comprises (a1) substantially amorphous telmisartan and (a2) alkaline agent, and the extragranular phase comprises (b1) a combination of amlodipine, hydrochlorothiazide and indapamide and (b2) disintegrant. In this embodiment, the extragranular phase preferably comprises amlodipine, hydrochlorothiazide and indapamide as the sole antihypertensive agents. The amlodipine, the hydrochlorothiazide and the indapamide are preferably all crystalline.

The total content of the one or more other antihypertensive agents (i.e. the antihypertensive agents other than telmisartan) in the monolayer tablet is typically 0.1 to 20% by weight, preferably 0.1 to 15% by weight, and more preferably 0.1 to 10% by weight, based on the total weight of the monolayer tablet. In a preferred embodiment, the other antihypertensive agents are solely contained in the extragranular phase, which means that none of the three APIs is additionally present in the intragranular phase. In the latter case, the total content of the one or more other antihypertensive agents in the extragranular phase is typically 0.1 to 15% by weight, preferably 0.2 to 10% by weight, and more preferably 0.5 to 5% by weight, based on the total weight of the monolayer tablet.

Among the aforementioned other antihypertensive agents, the content of amlodipine is typically 0.1 to 10% by weight, preferably 0.1 to 5% by weight, and more preferably 0.5 to 5% by weight, based on the total weight of the monolayer tablet. The content of hydrochlorothiazide is typically 0.1 to 15% by weight, preferably 0.1 to 10% by weight, and more preferably 1 to 5% by weight, based on the total weight of the monolayer tablet. The content of indapamide is typically 0.1 to 10% by weight, preferably 0.1 to 5% by weight, and more preferably 0.1 to 2% by weight, based on the total weight of the monolayer tablet.

The extragranular phase further comprises disintegrant as component (b2). The at least one disintegrant aims at facilitating breakage of the monolayer tablet into smaller fragments upon contact with a liquid such as water. The disintegrant can be suitably selected by a person skilled in the art and comprises, for example, agar, alginic acid and salts thereof, carrageenan, corn starch, crosslinked sodium carboxymethyl cellulose (sodium croscarmellose), crosslinked polyvinylpyrrolidone (crospovidone), pregelatinized starch, sodium carboxymethyl starch, sodium starch glycolate, and combinations thereof.

In a preferred embodiment, the disintegrant comprises crospovidone, sodium croscarmellose or/and sodium starch glycolate. More preferably, the extragranular phase comprises crospovidone or/and sodium croscarmellose, with sodium croscarmellose being particularly preferred and providing an especially pronounced disintegrating effect.

The total content of disintegrant in the monolayer tablet is typically 1 to 30% by weight, preferably 5 to 25% by weight, and more preferably 7 to 20% by weight, based on the total weight of the monolayer tablet. In a preferred embodiment, disintegrant is solely contained in the extragranular phase, which means that the one or more disintegrants are not additionally present in the intragranular phase. In the latter case, the content of disintegrant in the extragranular phase is typically 1 to 30% by weight, preferably 5 to 25% by weight, and more preferably 7 to 20% by weight, based on the total weight of the monolayer tablet.

The intragranular phase or/and the extragranular phase may comprise, in addition to the aforementioned mandatory components, one or more pharmaceutically acceptable excipients other than alkaline agent and disintegrant. Such pharmaceutically acceptable excipients are known to a person skilled in the art and comprise, for instance, binders, coloring agents, fillers, flavors, glidants, lubricants, preservatives, surfactants and sweeteners as disclosed e.g. in US 2006/0045865 A1 and "Lexikon der Hilfsstoffe für Pharmazie, Kosmetik und angrenzende Gebiete" (5th Edition, edited by H.P. Fiedler, Editio Cantor Verlag, Aulendorf, 2001). In a preferred embodiment, the pharmaceutically acceptable excipients are selected from binders, coloring agents, fillers, glidants, lubricants and wicking agents.

In a preferred embodiment, the monolayer tablet further comprises binder. The at least one binder ensures that the tablet can be formed with the required mechanical strength and is preferably added to the intragranular phase as component (a3). Specific examples of suitable binders are known to a person skilled in the art and comprise cellulose derivates such as hydroxymethylcellulose, hydroxyethylcellulose and hydroxypropylcellulose, polyvinylpyrrolidone (povidone), starch, and combinations thereof. More preferably, the binder comprises polyvinylpyrrolidone.

The total content of binder in the monolayer tablet is typically 0.1 to 15% by weight, preferably 0.5 to 10% by weight, and more preferably 0.5 to 5% by weight, based on the total weight of the monolayer tablet. In a preferred embodiment, binder is solely contained in the intragranular phase, which means that the one or more binders are not additionally present in the extragranular phase. In the latter case, the content of binder in the intragranular phase is typically 0.1 to 15% by weight, preferably 0.5 to 10% by weight, and more preferably 0.5 to 5% by weight, based on the total weight of the monolayer tablet.

In a preferred embodiment, the monolayer tablet further comprises fillers (also called diluent). The at least one filler increases the bulk volume and weight of low-dose drugs to a limit at which a pharmaceutical dosage form can be formed (and also ensures sufficient disintegration of the tablet) and is preferably added to the intragranular phase as component (a4). Specific examples of fillers comprise celluloses, such as microcrystalline cellulose, monosaccharides, such as glucose, oligosaccharides, such as lactose and sucrose, sugar alcohols, such as mannitol, sorbitol and xylitol, calcium hydrogen phosphate, and combinations thereof. More preferably, the filler comprises a calcium hydrogen phosphate, cellulose or/and a sugar alcohol, with a combination of microcrystalline cellulose and a sugar alcohol being particularly preferred.

The total content of filler in the monolayer tablet is typically 10 to 45% by weight, preferably 15 to 40% by weight, and more preferably 20 to 35% by weight, based on the total weight of the monolayer tablet. In a preferred embodiment, filler is solely contained in the intragranular phase, which means that the one or more fillers are not additionally present in the extragranular phase. In the latter case, the content of filler in the intragranular phase is typically 10 to 45% by weight, preferably 15 to 40% by weight, and more preferably 20 to 35% by weight, based on the total weight of the monolayer tablet.

In a preferred embodiment, the monolayer tablet further comprises wicking agent. The at least one wicking agent ensures that a liquid such as water or an aqueous fluid, by being drawn into the wicking agent's network, is carried to surfaces inside the tablet and thereby creates channels or a network of increased surface area. The wicking agent is preferably added to the extragranular phase as component (b3). Specific examples of wicking agents comprise alumina, bentonite, calcium hydrogen phosphate, microcrystalline cellulose, kaolin, kaolin, magnesium aluminum silicate, niacinamide, polyester, polyethylene, m-pyrol, colloidal silicon dioxide, fumed silicon dioxide, sodium lauryl sulfate, titanium dioxide, and combinations thereof. More preferably, the wicking agent comprises calcium hydrogen phosphate or/and microcrystalline cellulose.

The total content of wicking agent in the monolayer tablet is typically 10 to 60% by weight, preferably 15 to 50% by weight, and more preferably 20 to 45% by weight, based on the total weight of the monolayer tablet. In a preferred embodiment, wicking agent is solely contained in the extragranular phase, which means that the one or more wicking agents are not additionally present in the intragranular phase. In the latter case, the content of wicking agent in the extragranular phase is typically 10 to 60% by weight, preferably 15 to 50% by weight, and more preferably 20 to 45% by weight, based on the total weight of the monolayer tablet.

In a preferred embodiment, the monolayer tablet further comprises lubricant. The at least one lubricant ensures that tablet formation and ejection can occur with low friction between the solid and the die wall and is preferably added to the extragranular phase as component (b4). Specific examples of suitable lubricants comprise anhydrous colloidal silica, fatty acids, such as stearic acid, fatty acids salts, such as magnesium stearate, sodium stearyl fumarate and glycerol behenate, and combinations thereof. More preferably, the lubricant comprises anhydrous colloidal silica or a stearic acid salt such as magnesium stearate or sodium stearyl fumarate.

The total content of lubricant in the monolayer tablet is typically 0.1 to 15% by weight, preferably 0.1 to 10% by weight, and more preferably 0.1 to 5% by weight, based on the total weight of the monolayer tablet. In a preferred embodiment, lubricant is solely contained in the extragranular phase, which means that the one or more lubricants are not additionally present in the intragranular phase. In the latter case, the content of lubricant in the extragranular phase is typically 0.1 to 15% by weight, preferably 0.1 to 10% by weight, and more preferably 0.1 to 5% by weight, based on the total weight of the monolayer tablet.

In a preferred embodiment, the intragranular phase or/and the extragranular phase does not comprise a buffering agent. More preferably, the intragranular phase is devoid of buffering agent. Specific examples of buffering agents comprise 3-{[tris(hydroxymethyl)methyl]amino}propanesulfonic acid, N,N-bis(2-hydroxyethyl)glycine, tris(hydroxymethyl)methylamin, N-tris(hydroxymethyl)methylglycine, 4-(2-hydroxyethyl)-1-piperazine ethanesulfonic acid, 2-{[tris(hydroxymethyl)methyl]amino}ethanesulfonic acid, 3-(N-morpholine)propanesulfonic acid, piperazine-N,N'-bis(2-ethanesulfonic acid), 2-(N-morpholine)ethanesulfonic acid, malic acid, citric acid and potassium citrate.

In a preferred embodiment, the intragranular phase or/and the extragranular phase does not comprise an aluminum magnesium silicate compound. More preferably, the intragranular phase of the monolayer tablet of the invention is devoid of an aluminum magnesium silicate compound. Specific examples of an aluminum magnesium silicate compound comprise magnesium aluminosilicate, magnesium aluminometasilicate and magnesium aluminum silicate, but are not limited thereto. Particularly preferably, the intragranular phase neither comprises a buffering agent nor an aluminum magnesium silicate compound as defined above.

It lies in the nature of pharmaceutically acceptable excipients that they sometimes can perform more than one function in a pharmaceutical formulation. That is, due to the nature of pharmaceutically acceptable excipients, it cannot be excluded that a certain substance meets the requirements of more than one of the components (a2), (a3), (a4), (b2), (b3) and (b4). In the context of this specification, however, the fiction preferably applies that a substance which is used as a particular pharmaceutical excipient is not simultaneously used as another pharmaceutical excipient, except for component (a4) and component (b3) where the same excipient might be used. For example, calcium hydrogen phosphate and microcrystalline cellulose can be used both as component (a4) and as component (b3).

If calcium hydrogen phosphate or microcrystalline cellulose is chosen both as filler (a4) and as wicking agent (b3), however, the amount of microcrystalline cellulose acting as a filler in the intragranular phase of the monolayer tablet does not contribute to the total content of wicking agent contained in the extragranular phase of the monolayer tablet, and the amount of microcrystalline cellulose acting as a wicking agent in the extragranular phase of the monolayer tablet does not contribute to the total content of filler contained in the intragranular phase of the monolayer tablet.

Based on the aforementioned definitions, the components comprised by the intragranular phase of the monolayer tablet can be suitably combined in accordance with needs. In a preferred embodiment, however, the monolayer tablet comprises (a) an intragranular phase comprising (a1) 2 to 25% by weight of amorphous telmisartan, (a2) 1 to 15% by weight of alkaline agent, (a3) 0.5 to 10% by weight of binder and (a4) 15 to 40% by weight of filler, based in each case on the total weight of the monolayer tablet.

Similarly, the components comprised by the extragranular phase of the monolayer tablet can be suitably combined by a person skilled in the art. In a preferred embodiment, the monolayer tablet comprises (b) an extragranular phase comprising (b1) 0.1 to 15% by weight of the other antihypertensive agents, (b2) 5 to 25% by weight of disintegrant, (b3) 15 to 50% by weight of wicking agent and (b4) 0.1 to 10% by weight of lubricant, based in each case on the total weight of the monolayer tablet.

In a preferred embodiment, the monolayer tablet of the invention comprises (a) an intragranular phase comprising (a1) 2 to 25% by weight of amorphous telmisartan, (a2) 1 to 15% by weight of alkaline agent, (a3) 0.5 to 10% by weight of binder and (a4) 15 to 40% by weight of filler, and (b) an extragranular phase comprising (b1) 0.1 to 15% by weight of the other antihypertensive agents, (b2) 5 to 25% by weight of disintegrant, (b3) 15 to 50% by weight of wicking agent and (b4) 0.1 to 10% by weight of lubricant, based in each case on the total weight of the monolayer tablet.

As pointed out above, the monolayer tablet of the invention comprises an extragranular phase which may contain amlodipine, hydrochlorothiazide or/and indapamide as antihypertensive agent(s). In such case, it is preferred that amlodipine, hydrochlorothiazide and indapamide are present in crystalline form and are added in amounts as proposed hereinbefore. In a preferred embodiment, the antihypertensive agents other than telmisartan thus comprise (b1-1) 0.5 to 5% by weight of crystalline amlodipine, (b1-2) 0.1 to 2% by weight of crystalline indapamide, or/and (b1-3) 1 to 10% by weight of crystalline hydrochlorothiazide, based in each case on the total weight of the monolayer tablet.

In a preferred embodiment, the antihypertensive agents other than telmisartan comprise (b1-1) 0.5 to 5% by weight of crystalline amlodipine, or/and (b1-2) 0.1 to 2% by weight of crystalline indapamide, based in each case on the total weight of the monolayer tablet. More preferably, the antihypertensive agents other than telmisartan comprise both (b1-1) 0.5 to 5% by weight of crystalline amlodipine and (b1-2) 0.1 to 2% by weight of crystalline indapamide, based in each case on the total weight of the monolayer tablet.

Since the monolayer tablet of the invention is intended for medical use, it will typically contain the two or more APIs in an amount that is sufficient to effectively treat a disease or pathological condition. For this purpose, it is preferred that the monolayer tablet comprises (a1) 40 to 80 mg of telmisartan (in terms of free acid), (b1-1) 5 to 10 mg of amlodipine (in terms of free base), (b1-2) 1.5 to 2.5 mg of indapamide (in terms of free base), or/and (b1-3) 12.5 to 25 mg of hydrochlorothiazide (in terms of free base).

In a preferred embodiment, the monolayer tablet comprises (a1) 40 to 80 mg of telmisartan (in terms of free acid), (b1-1) 5 to 10 mg of amlodipine (in terms of free base), or/and (b1-2) 1.5 to 2.5 mg of indapamide (in terms of free base).

In another preferred embodiment, the monolayer tablet comprises (a1) 40 to 80 mg of telmisartan (in terms of free acid), (b1-1) 5 to 10 mg of amlodipine (in terms of free base), and (b1-2) 1.5 to 2.5 mg of indapamide (in terms of free base).

In still another preferred embodiment, the monolayer tablet comprises (a1) 40 to 80 mg of telmisartan (in terms of free acid), (b1-1) 5 to 10 mg of amlodipine (in terms of free base), and (b1-2) 2.5 mg of indapamide (in terms of free base).

The monolayer tablet of the invention has the two or more APIs combined in a single layer, so as to simplify the administration of the APIs and to increase patient compliance. Examples of tablets suitable for use in the invention comprise, but are not limited to, chewable tablets, coated tablets, effervescent tablets, lozenges, non-coated tablets and orally disintegrating tablets. In a preferred embodiment, however, the monolayer tablet is formulated for peroral delivery, and is preferably a non-coated or coated tablet formulated for peroral delivery. If a coated tablet is used, the coating is preferably a film-coating that does not affect immediate release of the APIs and typically has a thickness of 10 to 100 µm, preferably 20 to 70 µm.

In order to achieve a quick therapeutic response, the monolayer tablet is additionally formulated as an immediate release monolayer tablet ensuring immediate release of the two or more APIs upon administration to a patient. In this context, the term "immediate release" (IR) as used herein designates a release profile of an API which, upon subjecting the monolayer tablet for 30 minutes to an *in vitro* dissolution test in accordance with Pharm. Eur. 11.0, Chapter 2.9.3 (basket apparatus, 20 mesh size, 100 rpm, 900 ml of 0.01 hydrochloric acid (HCl) at 37°C), correlates with a release of at least 60% by weight, preferably at least 70% by weight, and more preferably at least 80% by weight of the API, based on the total amount of the API contained in the monolayer tablet.

In a preferred embodiment, the monolayer tablet is thus formulated in such a manner that at least 70% by weight of telmisartan, based on the total amount of telmisartan contained in the monolayer tablet, are released upon subjecting the monolayer tablet for 30 minutes to an *in vitro* dissolution test in accordance with Pharm. Eur. 11.0, Chapter 2.9.3, basket apparatus, 20 mesh size, 100 rpm, 900 ml of 0.01 HCl at 37°C. Particularly preferably, at least 80% or at least 90% by weight of telmisartan are released within the 30 minutes testing period.

In another preferred embodiment, the monolayer tablet is formulated in such a manner that at least 70% by weight of amlodipine, based on the total amount of amlodipine contained in the monolayer tablet, are released upon subjecting the monolayer tablet for 30 minutes to an *in vitro* dissolution test in accordance with Pharm. Eur. 11.0, Chapter 2.9.3, basket apparatus, 20 mesh size, 100 rpm, 900 ml of 0.01 HCl at 37°C. More preferably, at least 80% or at least 90% by weight of amlodipine are released within the 30 minutes testing period.

In another preferred embodiment, the monolayer tablet is formulated in such a manner that at least 70% by weight of hydrochlorothiazide, based on the total amount of hydrochlorothiazide contained in the monolayer tablet, are released upon subjecting the monolayer tablet for 30 minutes to an *in vitro* dissolution test in accordance with Pharm. Eur. 11.0, Chapter 2.9.3, basket apparatus, 20 mesh size, 100 rpm, 900 ml of 0.01 HCl at 37°C. More preferably, at least 80% by weight of hydrochlorothiazide are released within the 30 minutes testing period.

In another preferred embodiment, the monolayer tablet is formulated in such a manner that at least 70% by weight of indapamide, based on the total amount of indapamide contained in the monolayer tablet, are released upon subjecting the monolayer tablet for 30 minutes to an *in vitro* dissolution test in accordance with Pharm. Eur. 11.0, Chapter 2.9.3, basket apparatus, 20 mesh size, 100 rpm, 900 ml of 0.01 HCl at 37°C. More preferably, at least 80% by weight of indapamide are released within the 30 minutes testing period.

In another preferred embodiment, the monolayer tablet is formulated in such a manner that at least 70% by weight of each of telmisartan, amlodipine and indapamide, based on the total amounts of telmisartan, amlodipine and indapamide contained in the monolayer tablet, are released upon subjecting the monolayer tablet for 30 minutes to an *in vitro* dissolution test in accordance with Pharm. Eur. 11.0, Chapter 2.9.3, basket apparatus, 20 mesh size, 100 rpm, 900 ml of 0.01 HCl at 37°C. More preferably, at least 80% by weight of each API are released within the 30 minutes testing period.

In a preferred embodiment, the monolayer tablet further exhibits one or more of the following properties:
- a content uniformity of 85 to 115%, preferably 95 to 110%, and more preferably 95 to 105%, as determined in accordance with Ph. Eur., 11.0, Chapter 2.9.6;
- a friability of less than 5%, preferably less than 2%, and more preferably less than 1%, as determined in accordance with Ph. Eur., 11.0, Chapter 2.9.7; and
- a hardness of 40 to 300 N, preferably 50 to 280 N, and more preferably 60 to 200 N, as measured in accordance with Ph. Eur., 11.0, Chapter 2.9.8.

The monolayer tablet of the invention can basically be produced by any method allowing a person skilled in the art to manufacture a tablet comprising an intragranular phase and an extragranular phase as defined hereinbefore by suitably selecting the mandatory and optional components in accordance with needs, and subsequently processing those components using well-known granulation and tableting compression techniques. In a preferred embodiment, however, the monolayer tablet of the invention is manufactured by the method described hereinafter.

In another broad aspect, the invention relates to a method of manufacturing a monolayer tablet for immediate release, and to a monolayer tablet for immediate release which is obtainable by this method. The method comprises the steps of:
(i) wet-granulating telmisartan in the presence of alkaline agent, filler and optionally further pharmaceutically acceptable excipients so as to obtain telmisartan-containing granules;
(ii) mixing the telmisartan-containing granules with at least one antihypertensive agent selected from amlodipine, hydrochlorothiazide and indapamide, disintegrant and optionally further pharmaceutically acceptable excipients so as to obtain a blend;
(iii) compressing the blend so as to obtain an immediate release monolayer tablet; and
(iv) optionally coating the immediate release monolayer tablet.

Step (i) of the manufacturing method of the invention comprises wet-granulating telmisartan in the presence of alkaline agent, filler and optionally further pharmaceutically acceptable excipients. For this purpose, telmisartan (preferably in the form of free acid and typically in crystalline form), alkaline agent, filler and optionally other pharmaceutically acceptable excipients as described hereinbefore in connection with the monolayer tablet of the invention are provided in suitable amounts (e.g. in amounts of 1 to 30% by weight of telmisartan, 0.2 to 20% by weight of alkaline agent and 10 to 45% by weight of filler, based on the total weight of the final monolayer tablet), and are then wet-granulated using a granulation liquid.

The granulation liquid preferably comprises a binder as described hereinbefore in connection with the monolayer tablet of the invention, which binder is preferably homogeneously distributed within a suitable solvent. Suitable solvents for preparing the granulation liquid comprise, for example, water, alcohols, such as ethanol, n-propanol and isopropanol, and combinations thereof, with water being particularly preferred. For wet-granulation, the mixture of step (i) is brought into contact with the granulation liquid. In this context, the granulation liquid is typically sprayed on other dry excipients at an inlet air temperature of 20 to 100°C, preferably 40 to 80°C.

As known by a person skilled in the art, wet-granulation can, for example, be carried out using a high-speed mixer/granulator, a fluidized-bed granulator, a spray drier, or in accordance with the pellet-layering method. In a high-speed mixer/granulator, the components to be granulated are first blended and then contacted with the granulation liquid (which is added via a port while the impeller is rotating). The wetted mass can further be chopped and sieved, and the resulting granules are dried. In a spray dryer, the components are first dissolved or suspended in a liquid, and then the solution or suspension is sprayed into a heated and filtered airstream so as to coagulate the particles.

In a preferred embodiment, step (i) comprises fluid-bed granulating telmisartan using a fluidized-bed granulator. In a fluidized-bed granulator, the components to be granulated are fluidized in a heated and filtered airstream. A granulation liquid can be sprayed onto these "fluidized" particles by one or more spray nozzles. After the granulation process, the feed charge of heated and filtered air is continued and the granules are dried. In this embodiment, the granulation liquid comprises an alkaline agent as described hereinbefore in connection with the monolayer tablet of the invention.

More preferably, step (i) comprises fluid-bed granulating telmisartan, wherein telmisartan is dissolved in a granulation liquid comprising the alkaline agent, e.g. an alkaline metal hydroxide or/and a basic sugar alcohol. In this context, a granulation liquid having both components dissolved in an aqueous solvent such as water is particularly preferred. In order to achieve sufficient dissolution of telmisartan, the granulation liquid normally has a pH of 9.0 to 14.0. Preferably, the granulation liquid has a pH of 10.0 to 14.0, more preferably 11.0 to 13.0. Typically, such granulation liquid is then sprayed onto fluidized particles, e.g. particles derived from filler as described in connection with the monolayer tablet of the invention. As filler, a combination of microcrystalline cellulose and a sugar alcohol is particularly preferred.

After the wet-granulation step, a drying step can optionally be adopted. Drying generally takes place in conventional drying equipment, such as tray dryers, vacuum dryers, fluidized-bed dryers, spray dryers or freeze dryers. According to the invention, however, the wet-granulation step and the drying step are preferably performed in one and the same apparatus. The dried granules may further be screened so as to have a predetermined particle size distribution. The telmisartan-containing granules obtained in step (i) typically have a volume average particle size of 50 to 450 µm, as measured by dynamic image analysis. In a preferred embodiment, the telmisartan-containing granules have a volume average particle size of 100 to 400 µm, in particular 150 to 350 µm.

Step (ii) of the manufacturing method of the invention comprises mixing the telmisartan-containing granules with at least one antihypertensive agent selected from amlodipine, hydrochlorothiazide and indapamide, disintegrant and optionally further pharmaceutically acceptable excipients so as to obtain a blend. For this purpose, amlodipine (preferably in the form of a pharmaceutically acceptable salt), hydrochlorothiazide (preferably in the form of free base) or/and indapamide (preferably in the form of free base) are provided in suitable amounts (e.g. in amounts of 0.1 to 10% by weight of the respective API, based on the total weight of the final monolayer tablet), and are then mixed with the telmisartan-granules obtained in step (i), disintegrant and optionally other pharmaceutically acceptable excipients as described hereinbefore in connection with the monolayer tablet of the invention. Preferably, the telmisartan-containing granules are admixed with a combination of amlodipine and indapamide.

In this context, each of amlodipine, hydrochlorothiazide and indapamide is typically provided in crystalline form, wherein the amlodipine particles generally have a volume median particle size (d50) of ≤ 200 µm, preferably ≤ 150 µm, and more preferably ≤ 120 µm, as measured by laser diffraction analysis. The hydrochlorothiazide particles generally have a volume median particle size (d50) of ≤ 150 µm, preferably ≤ 100 µm, and more preferably ≤ 60 µm, as measured by laser diffraction analysis. The indapamide particles generally have a volume median particle size (d50) of ≤ 150 µm, preferably ≤ 100 µm, and more preferably ≤ 70 µm, as measured by laser diffraction analysis. For each API, the lower limit of the volume median particle size (d50) is typically ≥ 1 µm, preferably ≥ 5 µm, and more preferably ≥ 10 µm.

The aforementioned components can be sieved before being blended, e.g. with a sieve having a mesh size of 100 to 1200 µm. The mixing step itself can be carried out in accordance with conventional techniques, e.g. by dry-mixing the individual components using a conventional mixing device such as a compulsory mixer or a free-fall-mixer, so as to provide a blend which is preferably essentially homogeneous. Blending is carried out as necessary, e.g. for a period of 1 to 45 minutes, preferably 5 to 30 minutes. By carrying out the mixing step as suggested above, a blend having high flowability is obtained.

In one embodiment, the mixing step can be conducted in such a manner that only a part of the other antihypertensive agents or/and only a part of the pharmaceutically acceptable excipients is added to the telmisartan-containing granules of step (i) in a mixing device, for example in a high shear or tumbler mixer or container-mixing device. After this first mixing step, another part of the other antihypertensive agents or/and the pharmaceutically acceptable excipients can be added, which may be followed by another mixing step. This procedure can be repeated until the last part of the antihypertensive agents or/and the pharmaceutically acceptable excipients is used, preferably one to five times. What is more, one can also premix/triturate different antihypertensive agents with a part of the pharmaceutically acceptable excipients and add these premixes to telmisartan granulate afterwards. This kind of mixing can ensure an especially even distribution of active pharmaceutical ingredients.

Step (iii) of the manufacturing method of the invention comprises compressing the blend so as to obtain an immediate release monolayer tablet. For this purpose, the blend obtained in step (ii) is typically supplied to the die of a tablet press and subsequently compressed under conditions which are selected by a person skilled in the art in accordance with needs, e.g. under a load of 1 to 50 kN. In a preferred embodiment, the load is 3 to 25 kN, with a load of 7 to 25 kN being particularly preferred. Suitable tablet presses are commercially available, for instance, from Romaco Kilian GmbH (Germany), Fette Compacting GmbH (Germany), Korsch AG (Germany) and Riva S.A. (Argentina).

Step (iv) of the manufacturing method of the invention is optional and comprises coating the immediate release monolayer tablet, if necessary. For this purpose, the immediate release monolayer tablet obtained in step (iii) can be coated with a coating that does not affect immediate release of the two or more APIs, e.g. with a film-coating. Compounds for forming a film-coating are known to a person skilled in the art and comprise, for instance, modified celluloses, polymethacrylates, polyvinylpyrrolidone, polyvinyl acetate phthalate, shellack or/and zein. If a film-coating is applied, such film coating typically has a thickness of about 10 to 100 µm, preferably 20 to 70 µm.

In a separate aspect, the blend obtained in step (ii) can be used without compression. In an embodiment, the blend obtained in step (ii) is filled into dosage forms like sachets or capsules.

Hence, a subject of the invention is a dosage form, in particular a capsule or sachet, containing a blend, obtainable by
(i) wet-granulating telmisartan in the presence of alkaline agent, filler and optionally further pharmaceutically acceptable excipients so as to obtain telmisartan-containing granules; and
(ii) mixing the telmisartan-containing granules with at least one antihypertensive agent selected from amlodipine, hydrochlorothiazide and indapamide, disintegrant and optionally further pharmaceutically acceptable excipients.

All explanations made above for the preferred embodiment of the intragranular phase and extragranular phase (e.g. for amount, type, processing of compounds (a1) - (a4) and (b1) - (b4)) apply for the present aspect as well.

In another broad aspect, the invention relates to the use of the monolayer tablet described herein for treating essential hypertension or/and reducing the risk of development of a condition associated with essential hypertension. The term "essential hypertension" is known in the art and means that the hypertension has no obvious cause. The terms "treat" and "treatment" as used herein designate an eradication or an amelioration of essential hypertension (coinciding with a reduction of the systolic blood pressure (SBP) value or/and the diastolic blood pressure (DBP) value relative to the respective value before the start of treatment with the monolayer tablet), or of one or more symptoms associated with essential hypertension.

In order to achieve a therapeutic (i.e. preventive or curative) effect, the monolayer tablet can be administered once a day or twice a day, in particular once a day, to a hypertensive patient. Based on the definition given in the 2023 hypertension guidelines of the ESH (Mancia et al., "2023 ESH Guidelines for the management of arterial hypertension", J. Hypertension 41(12) (2023), 1874-2071), the term "hypertensive patient" as used herein designates a human patient who repeatedly experiences a sitting systolic blood pressure (SBP) value of ≥ 140 mmHg or/and a sitting diastolic blood pressure (DBP) value of ≥ 90 mmHg.

According to the invention, the monolayer tablet can be used at different stages of antihypertensive therapy or/and can address different patient groups, i.e. can be administered to naïve or pre-treated hypertensive patients. Thus, in one embodiment, the monolayer tablet can be used as a first-line antihypertensive therapy for a hypertensive patient, i.e. a hypertensive patient who has not received any antihypertensive therapy before (also called naïve hypertensive patient).

In another embodiment, the monolayer tablet can be used as a second-line antihypertensive therapy for a hypertensive patient who is a non-responder to a previous antihypertensive therapy, i.e. a hypertensive patient who has received a previous antihypertensive therapy but has not responded thereto. In this context, the term "non-responder" as used herein designates a patient whose sitting SBP value or/and sitting DBP value does not decrease by at least 5% after having received one or more antihypertensive agents once a day over a period of 6 weeks.

In still another embodiment, the monolayer tablet can be used as a substitution antihypertensive therapy for a hypertensive patient whose blood pressure is adequately controlled by a previous antihypertensive therapy, i.e. a hypertensive patient who has received a previous antihypertensive therapy and has suitably responded thereto by having their blood pressure lowered to a sitting SBP value of < 140 mmHg and at a sitting DBP value of < 90 mmHg.

Since hypertension is a condition in which the blood pressure in the arterial system is persistently and chronically elevated, the monolayer tablet is typically used for long-term treatment and is administered over a prolonged period of time. The administration period can be suitably chosen by a physician in accordance with needs, especially based on the grade of hypertension experienced by a hypertensive patient.

In general, the administration period will be in the range of from a few weeks, e.g. 4 weeks or 8 weeks, to more than 1 year. Typically, however, the fixed combination medicinal product is administered as long as it is well tolerated and achieves adequate control of the blood pressure, the term "adequate control" as used herein meaning that the blood pressure of the hypertensive patient is controlled at a sitting SBP value of < 140 mmHg and at a sitting DBP value of < 90 mmHg.

In a preferred embodiment, the monolayer tablet to be administered comprises:
(α) telmisartan at a dose of 40 mg (in terms of free acid) and amlodipine at a dose of 5 mg (in terms of free base);
(β) telmisartan at a dose of 40 mg (in terms of free acid) and amlodipine at a dose of 10 mg (in terms of free base);
(γ) telmisartan at a dose of 80 mg (in terms of free acid) and amlodipine at a dose of 5 mg (in terms of free base);
(δ) telmisartan at a dose of 80 mg (in terms of free acid) and amlodipine at a dose of 10 mg (in terms of free base);
(ε) telmisartan at a dose of 40 mg (in terms of free acid) and hydrochlorothiazide at a dose of 12.5 mg (in terms of free base);
(ζ) telmisartan at a dose of 40 mg (in terms of free acid) and hydrochlorothiazide at a dose of 25 mg (in terms of free base);
(η) telmisartan at a dose of 80 mg (in terms of free acid) and hydrochlorothiazide at a dose of 12.5 mg (in terms of free base);
(θ) telmisartan at a dose of 80 mg (in terms of free acid) and hydrochlorothiazide at a dose of 25 mg (in terms of free base);
(ι) telmisartan at a dose of 40 mg (in terms of free acid) and indapamide at a dose of 2.5 mg (in terms of free base);
(κ) telmisartan at a dose of 80 mg (in terms of free acid) and indapamide at a dose of 2.5 mg (in terms of free base);
(λ) telmisartan at a dose of 40 mg (in terms of free acid), amlodipine at a dose of 5 mg (in terms of free base) and hydrochlorothiazide at a dose of 12.5 mg (in terms of free base);
(µ) telmisartan at a dose of 40 mg (in terms of free acid), amlodipine at a dose of 5 mg (in terms of free base) and hydrochlorothiazide at a dose of 25 mg (in terms of free base);
(ν) telmisartan at a dose of 40 mg (in terms of free acid), amlodipine at a dose of 10 mg (in terms of free base) and hydrochlorothiazide at a dose of 12.5 mg (in terms of free base);
(ξ) telmisartan at a dose of 40 mg (in terms of free acid), amlodipine at a dose of 10 mg (in terms of free base) and hydrochlorothiazide at a dose of 25 mg (in terms of free base);
(ο) telmisartan at a dose of 80 mg (in terms of free acid), amlodipine at a dose of 5 mg (in terms of free base) and hydrochlorothiazide at a dose of 12.5 mg (in terms of free base);
(π) telmisartan at a dose of 80 mg (in terms of free acid), amlodipine at a dose of 5 mg (in terms of free base) and hydrochlorothiazide at a dose of 25 mg (in terms of free base);
(ρ) telmisartan at a dose of 80 mg (in terms of free acid), amlodipine at a dose of 10 mg (in terms of free base) and hydrochlorothiazide at a dose of 12.5 mg (in terms of free base);
(ς) telmisartan at a dose of 80 mg (in terms of free acid), amlodipine at a dose of 10 mg (in terms of free base) and hydrochlorothiazide at a dose of 25 mg (in terms of free base);
(σ) telmisartan at a dose of 40 mg (in terms of free acid), amlodipine a dose of 5 mg (in terms of free base) and indapamide at a dose of 2.5 mg (in terms of free base);
(τ) telmisartan at a dose of 80 mg (in terms of free acid), amlodipine at a dose of 5 mg (in terms of free base) and indapamide at a dose of 2.5 mg (in terms of free base);
(υ) telmisartan at a dose of 80 mg (in terms of free acid), amlodipine at a dose of 10 mg (in terms of free base) and indapamide at a dose of 2.5 mg (in terms of free base).

In this context, a monolayer tablet comprising (σ) telmisartan at a dose of 40 mg (in terms of free acid), amlodipine at a dose of 5 mg (in terms of free base), and indapamide at a dose of 2.5 mg (in terms of free base); (τ) telmisartan at a dose of 80 mg (in terms of free acid), amlodipine at a dose of 5 mg (in terms of free base), and indapamide at a dose of 2.5 mg (in terms of free base); or (υ) telmisartan at a dose of 80 mg (in terms of free acid), amlodipine at a dose of 10 mg (in terms of free base), and indapamide at a dose of 2.5 mg (in terms of free base) is particularly preferred.

Alternatively or additionally to the treatment of essential hypertension, the monolayer tablet can be used for reducing the risk of development of a condition associated with essential hypertension, i.e. of a condition that possibly develops when a hypertensive patient is not undergoing any antihypertensive therapy or receives an antihypertensive therapy that does not allow for adequate control of the blood pressure.

In a preferred embodiment, such a condition associated with essential hypertension is a cardiovascular event. More preferably, the condition associated with essential hypertension is a cardiovascular event selected from acute coronary syndrome, coronary artery disease, coronary artery bypass graft, hypertensive encephalopathy, myocardial infarction, percutaneous transluminal coronary revascularization, stroke, transient ischemic attack and unstable angina.

The invention is particularly defined by the following items:
1. A monolayer tablet for immediate release comprising
   (a) an intragranular phase comprising (a1) substantially amorphous telmisartan, and (a2) alkaline agent; and
   (b) an extragranular phase comprising (b1) one or more other antihypertensive agents selected from amlodipine, hydrochlorothiazide and indapamide, and (b2) disintegrant.
2. The monolayer tablet according to item 1, wherein at least 70% by weight of telmisartan, based on the total amount of telmisartan contained in the tablet, are released upon subjecting the tablet for 30 minutes to an *in vitro* dissolution test in accordance with Pharm. Eur. 11.0, Chapter 2.9.3, basket apparatus, 20 mesh size, 100 rpm, 900 ml of 0.01 HCl at 37°C.
3. The monolayer tablet according to item 1 or 2, wherein the intragranular phase comprises
   (a1) 2 to 25% by weight of amorphous telmisartan,
   (a2) 1 to 15% by weight of alkaline agent,
   (a3) 0.5 to 10% by weight of binder, and
   (a4) 15 to 40% by weight of filler,

   and wherein the extragranular phase comprises
      (b1) 0.1 to 15% by weight of the other antihypertensive agents,
      (b2) 5 to 25% by weight of disintegrant,
      (b3) 15 to 50% by weight of wicking agent, and
      (b4) 0.1 to 10% by weight of lubricant,
   based in each case on the total weight of the tablet.
4. The monolayer tablet according to any one of items 1 to 3, wherein the other antihypertensive agents comprise
   (b1-1) 0.5 to 5% by weight of crystalline amlodipine; or/and
   (b1-2) 0.1 to 2% by weight of crystalline indapamide,
   based in each case on the total weight of the tablet.
5. The monolayer tablet according to any one of items 1 to 4, wherein the tablet comprises
   (a1) 40 to 80 mg of telmisartan;
   (b1-1) 5 to 10 mg of amlodipine; or/and
   (b1-2) 1.5 to 2.5 mg of indapamide.
6. The monolayer tablet according to any one of items 1 to 5, wherein the alkaline agent (a2) comprises an alkaline metal hydroxide, a basic amino acid or/and a basic sugar alcohol, in particular a combination of sodium hydroxide and meglumine.
7. The monolayer tablet according to any one of items 1 to 6, wherein the disintegrant (b2) comprises sodium croscarmellose.
8. The monolayer tablet according to any one of items 3 to 7, wherein the wicking agent (b3) comprises microcrystalline cellulose.
9. The monolayer tablet according to any one of items 1 to 8, wherein the intragranular phase does not comprise a buffering agent.
10. The monolayer tablet according to item 9, wherein the buffering agent is selected from 3-{[tris(hydroxymethyl)methyl]amino}propanesulfonic acid, N,N-bis(2-hydroxyethyl)glycine, tris(hydroxymethyl)methylamin, N-tris(hydroxymethyl)methylglycine, 4-(2-hydroxyethyl)-1-piperazine ethanesulfonic acid, 2-{[tris(hydroxymethyl)methyl]amino}ethanesulfonic acid, 3-(N-morpholine)propanesulfonic acid, piperazine-N,N'-bis(2-ethanesulfonic acid), 2-(N-morpholine)ethanesulfonic acid, malic acid, citric acid and potassium citrate.
11. The monolayer tablet according to any one of items 1 to 10, wherein the intragranular phase does not comprise an aluminum magnesium silicate compound.
12. The monolayer tablet according to item 11, wherein the aluminum magnesium silicate compound is selected from magnesium aluminosilicate, magnesium aluminometasilicate and magnesium aluminum silicate.
13. The monolayer tablet according to any one of items 1 to 12, wherein the monolayer tablet has a hardness of 50 to 280 N, as measured in accordance with Ph. Eur., 11.0, Chapter 2.9.8.
14. A method of manufacturing a monolayer tablet for immediate release, the method comprising the steps of:
   (i) wet-granulating telmisartan in the presence of alkaline agent, filler and optionally further pharmaceutically acceptable excipients so as to obtain telmisartan-containing granules;
   (ii) mixing the telmisartan-containing granules with at least one antihypertensive agent selected from amlodipine, hydrochlorothiazide and indapamide, a disintegrant and optionally further pharmaceutically acceptable excipients so as to obtain a blend;
   (iii) compressing the blend so as to obtain a monolayer tablet for immediate release; and
   (iv) optionally coating the immediate release monolayer tablet.
15. The method according to item 14, wherein step (i) comprises fluid-bed granulation of telmisartan dissolved in a granulation liquid comprising the alkaline agent.
16. The method according to item 15, wherein the granulation liquid has a pH of 10.0 to 14.0.
17. The method according to any one of items 14 to 16, wherein the telmisartan-containing granules obtained in step (i) have a volume average particle size of 100 to 400 µm, in particular 150 to 350 µm, as measured by dynamic image analysis.
18. The method according to any one of items 14 to 17, wherein step (iii) comprises compressing the blend under a load of 3 to 25 kN.
19. A monolayer tablet for immediate release, obtainable by the method according to any one of items 14 to 18.
20. The monolayer tablet according to any one of items 1 to 13 or 19 for use in treating essential hypertension or/and reducing the risk of development of a condition associated with essential hypertension, wherein the monolayer tablet comprises:
   (α) telmisartan at a dose of 40 mg and amlodipine at a dose of 5 mg;
   (β) telmisartan at a dose of 40 mg and amlodipine at a dose of 10 mg;
   (γ) telmisartan at a dose of 80 mg and amlodipine at a dose of 5 mg;
   (δ) telmisartan at a dose of 80 mg and amlodipine at a dose of 10 mg;
   (ε) telmisartan at a dose of 40 mg and hydrochlorothiazide at a dose of 12.5 mg;
   (ζ) telmisartan at a dose of 40 mg and hydrochlorothiazide at a dose of 25 mg;
   (η) telmisartan at a dose of 80 mg and hydrochlorothiazide at a dose of 12.5 mg;
   (θ) telmisartan at a dose of 80 mg and hydrochlorothiazide at a dose of 25 mg;
   (ι) telmisartan at a dose of 40 mg and indapamide at a dose of 2.5 mg;
   (κ) telmisartan at a dose of 80 mg and indapamide at a dose of 2.5 mg;
   (λ) telmisartan at a dose of 40 mg, amlodipine at a dose of 5 mg and hydrochlorothiazide at a dose of 12.5 mg;
   (µ) telmisartan at a dose of 40 mg, amlodipine at a dose of 5 mg and hydrochlorothiazide at a dose of 25 mg;
   (ν) telmisartan at a dose of 40 mg, amlodipine at a dose of 10 mg and hydrochlorothiazide at a dose of 12.5 mg;
   (ξ) telmisartan at a dose of 40 mg, amlodipine at a dose of 10 mg and hydrochlorothiazide at a dose of 25 mg;
   (ο) telmisartan at a dose of 80 mg, amlodipine at a dose of 5 mg and hydrochlorothiazide at a dose of 12.5 mg;
   (π) telmisartan at a dose of 80 mg, amlodipine at a dose of 5 mg and hydrochlorothiazide at a dose of 25 mg;
   (ρ) telmisartan at a dose of 80 mg, amlodipine at a dose of 10 mg and hydrochlorothiazide at a dose of 12.5 mg;
   (ς) telmisartan at a dose of 80 mg, amlodipine at a dose of 10 mg and hydrochlorothiazide at a dose of 25 mg;
   (σ) telmisartan at a dose of 40 mg, amlodipine a dose of 5 mg and indapamide at a dose of 2.5 mg;
   (τ) telmisartan at a dose of 80 mg, amlodipine at a dose of 5 mg and indapamide at a dose of 2.5 mg;
   (υ) telmisartan at a dose of 80 mg, amlodipine at a dose of 10 mg and indapamide at a dose of 2.5 mg.

### EXAMPLES

The invention will now be explained in more detail based on the following examples which are not intended to limit the scope of the claims.

### Measurement methods

The crystallinity of telmisartan was determined by X-ray powder diffraction analysis using PANalytical X'Pert PRO powder diffractometer (MPD, Netherlands).

The volume median particle size (d50) of active pharmaceutical ingredients was determined by laser diffraction analysis using a Mastersizer particle size analyzer (Malvern Panalytical GmbH, Germany).

The volume average particle size of telmisartan granulate was determined by dynamic image analysis using a Camsizer XT particle size analyzer (Microtrac Retsch GmbH, Germany).

### Example 1: Preparation of monolayer tablets including alkaline agent by wet-granulation

Monolayer tablets comprising each of telmisartan, amlodipine and indapamide as antihypertensive agents were manufactured by dissolving crystalline telmisartan (in the form of free acid) together with binder in an aqueous granulation liquid having a pH of about 12, and spraying the granulation liquid obtained thereby onto a mixture of alkaline agent and filler using a fluidized-bed granulator (GEA Group AG, Germany). After drying, telmisartan-containing granules having a volume average particle size of about 50 to 450 µm, as measured by dynamic image analysis, were obtained.

The dried telmisartan-containing granules were subsequently mixed with crystalline amlodipine (in the form of amlodipine besylate), crystalline indapamide (in the form of free base), disintegrant and filler using a container-mixing device for about 15 to 20 minutes at room temperature so as to obtain a blend (first mixing), and afterwards lubricant was added and mixed for 2 more minutes to obtain lubricated blend (second mixing). The blend was introduced into the die of a rotary tablet press (Kilian) and compressed under a load of about 7 to 25 kN.

Tables 1-4 show exemplary formulations suitable for manufacturing monolayer tablets in accordance with the aforementioned method.

**Table 1**

| | Formulation 1 | Formulation 2 | Formulation 3 |
|---|---|---|---|
| Material | Amount per tablet [mg] | Amount per tablet [mg] | Amount per tablet [mg] |
| Telmisartan | 80 | 80 | 40 |
| Sodium hydoxide | 6.72 | 6.72 | 3.36 |
| Meglumine | 24 | 24 | 12 |
| Povidone | 12 | 12 | 6 |
| Microcrystalline cellulose | 72 | 72 | 36 |
| Mannitol | 60 | 60 | 30 |
| **Telmisartan-containing granules** | **254.72** | **254.72** | **127.36** |
| Amlodipine besylate | 13,87 | 6.935 | 6.935 |
| Indapamide | 2.5 | 2.5 | 2.5 |
| Sodium croscarmellose | 75 | 75 | 37.5 |
| Microcrystalline cellulose | 147.91 | 155.545 | 72.71 |
| Magnesium stearate | 5 | 5 | 2.5 |
| Anhydrous colloidal silicon dioxide | / | 2.5 | / |
| Iron oxide, yellow* | 1 | 1 | 1 |
| **Monolayer tablet** | **500.00** | **500.00** | **250.00** |
| **optional* | | | |

**Table 2**

| | Formulation 4 | Formulation 5 | Formulation 6 |
|---|---|---|---|
| Material | Amount per tablet [mg] | Amount per tablet [mg] | Amount per tablet [mg] |
| Telmisartan | 80 | 80 | 80 |
| Sodium hydoxide | 6.72 | 6.72 | 6.72 |
| Meglumine | 24 | 24 | 24 |
| Povidone | 12 | 12 | 12 |
| Microcrystalline cellulose | 72 | 132 | 132 |
| Mannitol | 60 | / | / |
| **Telmisartan-containing granules** | **254.72** | **254.72** | **254.72** |
| Amlodipine besylate | 13.87 | 13.87 | 13.87 |
| Indapamide | 2.5 | 2.5 | 2.5 |
| Sodium croscarmellose | 40 | 40 | 40 |
| Microcrystalline cellulose | 82.11 | 82.11 | / |
| Mannitol | / | / | 82.11 |
| Magnesium stearate | 4 | 4 | 4 |
| Anhydrous colloidal silicon dioxide | 2 | 2 | 2 |
| Iron oxide, yellow* | 0.8 | 0.8 | 0.8 |
| **Monolayer tablet** | **400.00** | **400.00** | **400.00** |

| | | | |
|---|---|---|---|
| **optional* | | | |

**Table 3**

| | Formulation 7 | Formulation 8 | Formulation 9 |
|---|---|---|---|
| Material | Amount per tablet [mg] | Amount per tablet [mg] | Amount per tablet [mg] |
| Telmisartan | 80 | 80 | 80 |
| Sodium hydoxide | 6.72 | 6.72 | 6.72 |
| Meglumine | 24 | 24 | / |
| Arginine | / | / | 24 |
| Povidone | 12 | 12 | 12 |
| Microcrystalline cellulose | 72 | 72 | 72 |
| Mannitol | 60 | 60 | 60 |
| **Telmisartan-containing granules** | **254.72** | **254.72** | **254.72** |
| Amlodipine besylate | 13.87 | 13.87 | 13.87 |
| Indapamide | 2.5 | 2.5 | 2.5 |
| Sodium croscarmellose | 75 | 75 | 75 |
| Microcrystalline cellulose | / | 100 | 100 |
| Calcium hydrogen phosphate | 147.91 | 47.91 | 47.91 |
| Magnesium stearate | 5 | 5 | 5 |
| Iron oxide, yellow* | 1 | 1 | 1 |
| **Monolayer tablet** | **500.00** | **500.00** | **500.00** |

| | | | |
|---|---|---|---|
| **optional* | | | |

**Table 4**

| | Formulation 10 | Formulation 11 | Formulation 12 |
|---|---|---|---|
| Material | Amount per tablet [mg] | Amount per tablet [mg] | Amount per tablet [mg] |
| Telmisartan | 80 | 80 | 80 |
| Sodium hydoxide | 6.72 | 6.72 | 6.72 |
| Meglumine | 24 | 24 | 24 |
| Povidone | 12 | 12 | 12 |
| Microcrystalline cellulose | 72 | 72 | 72 |
| Mannitol | 60 | 60 | 60 |
| **Telmisartan-containing granules** | **254.72** | **254.72** | **254.72** |
| Amlodipine besylate | 13.87 | 13.87 | 13.87 |
| Indapamide | 2.5 | 2.5 | 2.5 |
| Sodium starch glycolate | 75 | / | / |
| Crospovidone | / | 75 | 50 |
| Microcrystalline cellulose | 147.91 | 147.91 | 172.91 |
| Magnesium stearate | 5 | 5 | 5 |
| Iron oxide, yellow* | 1 | 1 | 1 |
| **Monolayer tablet** | **500.00** | **500.00** | **500.00** |
| **optional* | | | |

By manufacturing monolayer tablets from Formulations 1 to 12 in accordance with the method of Example 1 and adjusting the conditions of the compression step, monolayer tablets having a hardness of 181 N (optimal hardness) or a hardness of 253 N (high hardness) were obtained. The monolayer tablets having a hardness of 181 N had a disintegration time of about 8 to 9 minutes determined in accordance with Pharm. Eur. 11.0, Chapter 2.9.1.A, whereas the monolayer tablets having a hardness of 253 N were found to have a disintegration time of about 10 to 11 minutes. In both cases, friability was 0%.

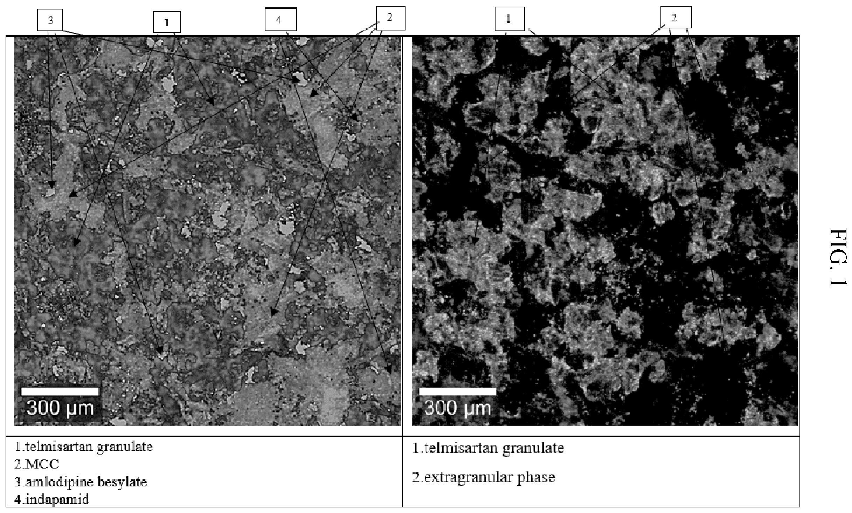

As can be deduced from the Raman microscope images of Fig. 1 (laser 532 nm, 20 × objective, analyzed surface 1500 × 1500 nm), the monolayer tablets manufactured in accordance with the method of Example 1 comprised an intragranular phase comprising substantially amorphous telmisartan, and an extragranular phase comprising, among others, amlodipine besylate, indapamide, sodium croscarmellose and microcrystalline cellulose.

### Comparative example 1: Preparation of monolayer tablets including alkaline agent by direct compression

Comparative monolayer tablets comprising each of telmisartan, amlodipine and indapamide as antihypertensive agents were manufactured in accordance with the method of Example 1, except for the fact that fluidized-bed granulation of telmisartan was omitted and a blend obtained by mixing 80 mg of telmisartan, 13.87 mg of amlodipine besylate, 2.5 mg of indapamide, 6.72 mg of sodium hydroxide, 24 mg of meglumine, 12 mg of Povidone, 218.41 mg of microcrystalline cellulose, 60 mg of mannitol, 75 mg of sodium croscarmellose, 5 mg of magnesium stearate and 5 mg of anhydrous colloidal silicon dioxide (corresponding to Formulation 1 of Example 1) was directly compressed.

By adjusting the conditions of the compression step, monolayer tablets having a hardness of 182 N (optimal hardness) or a hardness of 262 N (high hardness) were obtained. The monolayer tablets having a hardness of 182 N had a disintegration time of about 9 to 10 minutes, whereas the monolayer tablets having a hardness of 262 N were found to have a disintegration time of about 10 to 13 minutes.

### Comparative example 2: Preparation of monolayer tablets lacking alkaline agent by direct compression

Comparative monolayer tablets comprising each of telmisartan, amlodipine and indapamide as antihypertensive agents were manufactured in accordance with Example 15 of CN 101 450 211 A. For this purpose, 80 mg of telmisartan, 13.87 mg of amlodipine besylate, 2.5 mg of indapamide, 10 mg of anhydrous colloidal silicon dioxide, 95.13 mg of microcrystalline cellulose, 286 mg of pregelatinized starch (molecular weight 1500 g/mol), 10 mg of crospovidone and 2.5 mg of magnesium stearate were mixed so as to obtain a blend, and the blend was directly compressed. The mixing conditions and the tabletting conditions were the same as those described in Example 1.

By suitably adjusting the conditions of the compression step, monolayer tablets having a hardness of 182 N (optimal hardness) or a hardness of 202 N (high hardness) were obtained. The monolayer tablets having a hardness of 182 N had a disintegration time of about 2 to 3 minutes, whereas the monolayer tablets having a hardness of 202 N were found to have a disintegration time of about 3 to 4 minutes.

### Example 2: Evaluation of flowability

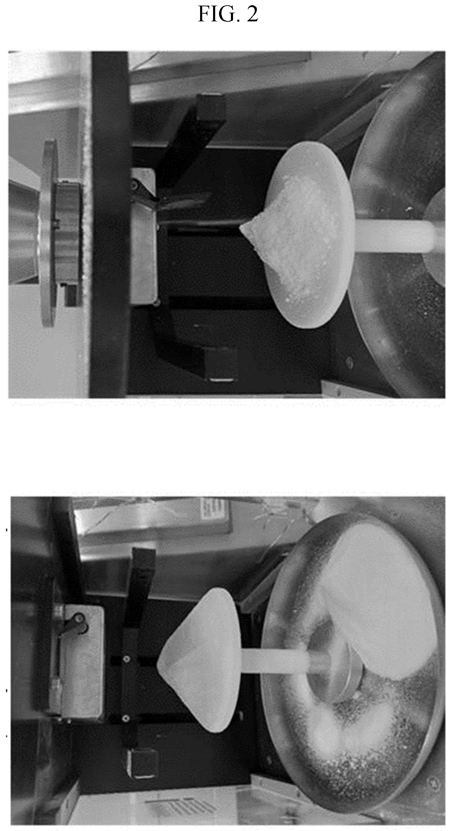

Flowability of the blends underlying the monolayer tablets manufactured in accordance with the methods of Example 1 and Comparative Example 1 was compared by visual inspection. As can be deduced from the left-hand side photograph of Fig. 2, the blend of Formulation 1 comprising granulated telmisartan exhibited excellent flowability, coinciding with good tabletting processability. In contrast thereto, the right-hand side photograph reveals that the blend of Comparative Example 1 having all three APIs directly added to the excipients had poor flowability and poor compactability. The very same deficiencies were also found for the blend of Comparative Example 2.

### Example 3: Evaluation of API stability

Stability of the APIs comprised by the monolayer tablets manufactured in accordance with the method of Example 1 was determined by subjecting the monolayer tablets to a long-term stress test at different temperatures and different levels of relative humidity (abbreviated as RH). In this context, Table 5 shows the stability data of telmisartan, amlodipine and indapamide in monolayer tablets derived from Formulation 1 and produced in a laboratory scale experiment.

**Table 5**

| | Telmisartan | | Amlodipine | | Indapamide | |
|---|---|---|---|---|---|---|
| Stress condition | Individual unspecified impurities [%] | Total [%] | Individual unspecified impurities [%] | Total [%] | Individual unspecified impurities [%] | Total [%] |
| t0 | ≤ 0.10 | ≤ 0.10 | ≤ 0.10 | ≤ 0.10 | ≤ 0.10 | ≤ 0.10 |
| 40°C, 75% RH, 6 months | /* | /* | ≤ 0.10 | ≤ 0.10 | ≤ 0.10 | ≤ 0.10 |

| | | | | | | |
|---|---|---|---|---|---|---|
| ** telmisartan not critical for stability and not analyzed at every time spot* | | | | | | |

Table 6 shows the stability data of telmisartan, amlodipine and indapamide in monolayer tablets derived from Formulation 1 and produced in a scale-up experiment.

**Table 6**

| | Telmisartan | | Amlodipine | | Indapamide | |
|---|---|---|---|---|---|---|
| Stress condition | Individual unspecified impurities [%] | Total [%] | Individual unspecified impurities [%] | Total [%] | Individual unspecified impurities [%] | Total [%] |
| t0 | ≤ 0.10 | ≤ 0.10 | ≤ 0.10 | ≤ 0.10 | ≤ 0.10 | ≤ 0.10 |
| 25°C, 60% RH, 3 months | ≤ 0.10 | ≤ 0.10 | ≤ 0.10 | ≤ 0.10 | ≤ 0.10 | ≤ 0.10 |
| 40°C, 75% RH, 3 months | ≤ 0.10 | ≤ 0.10 | ≤ 0.10 | ≤ 0.10 | ≤ 0.10 | ≤ 0.10 |

As can be deduced from Tables 5 and 6, amlodipine and indapamide are stable over a long period of time even when subjected to temperatures well above room temperature or/and to high relative humidity. Accordingly, it is understood that, in the monolayer tablets manufactured by the method of Example 1, the alkaline environment provided by the telmisartan-containing granules does not affect stability of the other antihypertensive agents, and in particular the stability of amlodipine.

### Example 4: Evaluation of dissolution properties

Dissolution properties of the monolayer tablets manufactured in accordance with the methods of Example 1, Comparative Example 1 and Comparative Example 2 were compared. For this purpose, the monolayer tablets were subjected for 45 minutes to an *in vitro* dissolution test in accordance with Pharm. Eur. 11.0, Chapter 2.9.3 (basket apparatus, 20 mesh size, 100 rpm, 900 ml of 0.01 HCl at 37°C).

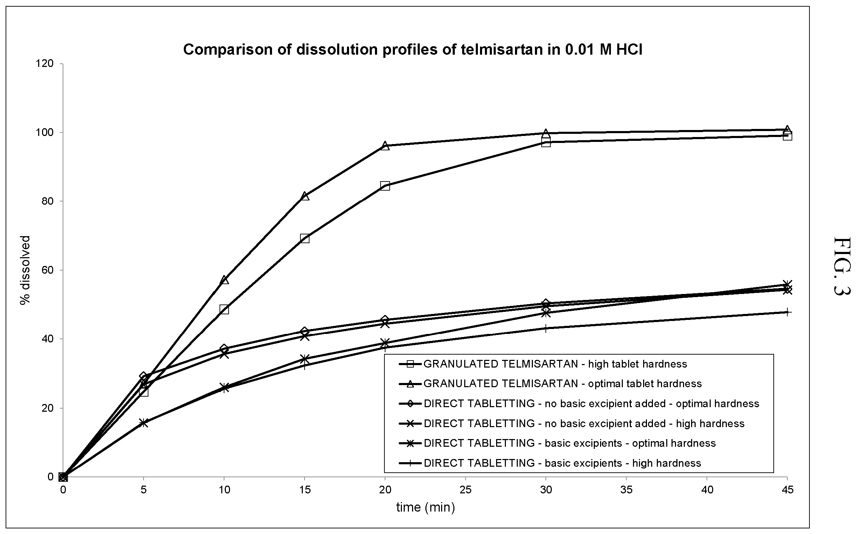

As can be deduced from the diagram of Fig. 3, monolayer tablets manufactured in accordance with the method of Example 1 and derived from Formulation 1 ("granulated telmisartan") released more than 95% by weight of telmisartan, based on the total amount of telmisartan contained in the monolayer tablet, within 30 minutes of exposure. In contrast thereto, monolayer tablets manufactured in accordance with the method of Comparative Examples 1 and 2 ("direct tabletting" with or without alkaline agent) released less than 50% by weight of telmisartan within the same time period.

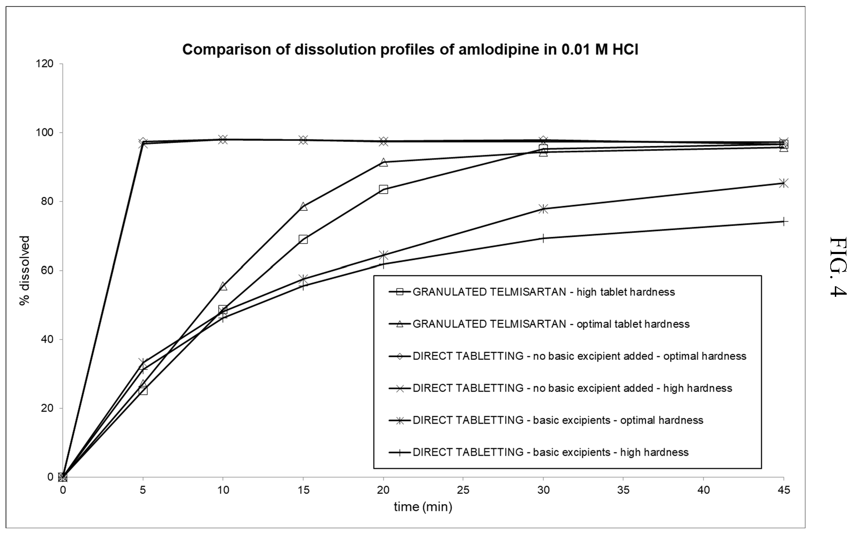

Furthermore, the diagram of Fig. 4 provides evidence that monolayer tablets manufactured in accordance with the method of Example 1 and derived from Formulation 1 ("granulated telmisartan") released more than 90% by weight of amlodipine, based on the total amount of amlodipine contained in the monolayer tablet, within 30 minutes of exposure. In contrast thereto, monolayer tablets manufactured in accordance with the method of Comparative Example 1 ("direct tabletting" with alkaline agent) released less than 80% by weight of amlodipine within the same time period.

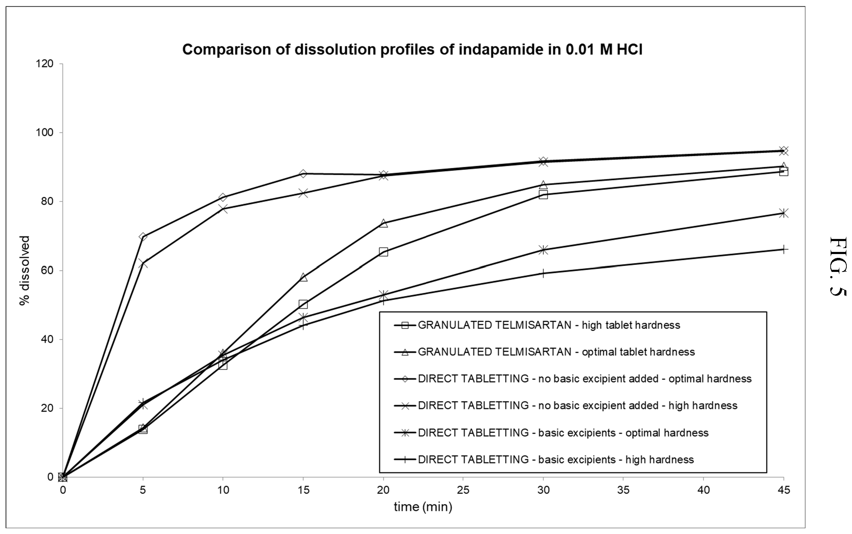

Finally, the diagram of Fig. 5 shows that monolayer tablets manufactured in accordance with the method of Example 1 and derived from Formulation 1 ("granulated telmisartan") released more than 80% by weight of indapamide, based on the total amount of indapamide contained in the monolayer tablet, within 30 minutes of exposure. In contrast thereto, monolayer tablets manufactured in accordance with the method of Comparative Example 1 ("direct tabletting" with alkaline agent) released less than 70% by weight of indapamide within the same time period.

In view of the foregoing it is understood that monolayer tablets manufactured in accordance with the method of Example 1 have immediate release properties and ensure immediate release of each of telmisartan, amlodipine and indapamide. Moreover, it is shown that the release of telmisartan from the monolayer tablets of Example 1 is significantly faster than the release of telmisartan from the monolayer tablets of Comparative Examples 1 and 2, and that the release profile of amlodipine and indapamide in the monolayer tablets of Example 1 is similar to that of telmisartan. By this means, essentially simultaneous release of all three APIs can be ensured.

## Claims

1. A monolayer tablet for immediate release comprising
(a) an intragranular phase comprising (a1) substantially amorphous telmisartan, and (a2) alkaline agent; and
(b) an extragranular phase comprising
(b1) another antihypertensive agent selected from amlodipine, hydrochlorthiazide, indapamide or a combination of amlodipine and hydrochlorothiazide, and
(b2) disintegrant,
wherein each of the telmisartan and one or more of the other antihypertensive agents may be present in the form of the active pharmaceutical ingredient as such, in the form of a pharmaceutically acceptable salt, solvate or salt solvate of the active pharmaceutical ingredient.

2. The monolayer tablet according to claim 1, wherein at least 70% by weight of telmisartan, based on the total amount of telmisartan contained in the tablet, are released upon subjecting the tablet for 30 minutes to an *in vitro* dissolution test in accordance with Pharm. Eur. 11.0, Chapter 2.9.3, basket apparatus, 20 mesh size, 100 rpm, 900 ml of 0.01 HCl at 37°C.

3. The monolayer tablet according to claim 1 or 2, wherein the intragranular phase comprises
(a1) 2 to 25% by weight of amorphous telmisartan,
(a2) 1 to 15% by weight of alkaline agent,
(a3) 0.5 to 10% by weight of binder, and
(a4) 15 to 40% by weight of filler,
and wherein the extragranular phase comprises
(b1) 0.1 to 15% by weight of the other antihypertensive agents,
(b2) 5 to 25% by weight of disintegrant,
(b3) 15 to 50% by weight of wicking agent, and
(b4) 0.1 to 10% by weight of lubricant,
based in each case on the total weight of the tablet.

4. The monolayer tablet according to any one of claims 1 to 3, wherein the other antihypertensive agents comprise
(b1) 0.1 to 10% by weight of crystalline indapamide; or
(b1) 0.1 to 10% by weight of crystalline amlodipine; or
(b1) 0.1 to 15% by weight of crystalline hydrochlorothiazide; or a combination of
(b1-1) 0.5 to 5% by weight of crystalline amlodipine; and
(b1-2) 1 to 10% by weight of crystalline hydrochlorothiazide,
based in each case on the total weight of the tablet.

5. The monolayer tablet according to any one of claims 1 to 4, wherein the tablet comprises
(a1) 40 to 80 mg of telmisartan and
(b1) 1.5 - 2.5 mg indapamide; or
(a1) 40 to 80 mg of telmisartan and
(b1) 5 to 10 mg of amlodipine; or
(a1) 40 to 80 mg of telmisartan and
(b1) 12.5 to 25 mg of hydrochlorothiazide; or
(a1) 40 to 80 mg of telmisartan and
(b1-1) 5 to 10 mg of amlodipine; and
(b1-2) 12.5 to 25 mg of hydrochlorothiazide.

6. The monolayer tablet according to any one of claims 1 to 5, wherein the alkaline agent (a2) comprises an alkaline metal hydroxide, a basic amino acid or/and a basic sugar alcohol, in particular a combination of sodium hydroxide and meglumine.

7. The monolayer tablet according to any one of claims 1 to 6, wherein the disintegrant (b2) comprises sodium croscarmellose.

8. The monolayer tablet according to any one of claims 3 to 7, wherein the wicking agent (b3) comprises microcrystalline cellulose.

9. The monolayer tablet according to any one of claims 1 to 8, wherein the intragranular phase does not comprise a buffering agent or/and an aluminum magnesium silicate compound.

10. The monolayer tablet according to any one of claims 1 to 9, wherein the monolayer tablet has a hardness of 50 to 280 N, as measured in accordance with Ph. Eur., 11.0, Chapter 2.9.8.

11. A method of manufacturing a monolayer tablet for immediate release, the method comprising the steps of:
(i) wet-granulating telmisartan in the presence of alkaline agent, filler and optionally further pharmaceutically acceptable excipients so as to obtain telmisartan-containing granules;
(ii) mixing the telmisartan-containing granules with amlodipine and hydrochlorothiazide or mixing the telmisartan-containing granules with either indapamide, amlodipine or hydrochlorothiazide, a disintegrant and optionally further pharmaceutically acceptable excipients so as to obtain a blend;
(iii) compressing the blend so as to obtain a monolayer tablet for immediate release; and
(iv) optionally coating the immediate release monolayer tablet,
wherein each of the telmisartan, the amlodipine, hydrochlorothiazide and/or the indapamide may be present in the form of the active pharmaceutical ingredient as such, in the form of a pharmaceutically acceptable salt, solvate or salt solvate of the active pharmaceutical ingredient.

12. The method according to claim 11, wherein step (i) comprises fluid-bed granulation of telmisartan dissolved in a granulation liquid comprising the alkaline agent.

13. The method according to claim 12, wherein the granulation liquid has a pH of 10.0 to 14.0.

14. The method according to any one of claims 11 to 13, wherein the telmisartan-containing granules obtained in step (i) have a volume average particle size of 100 to 400 µm, in particular 150 to 350 µm, as measured by dynamic image analysis.

15. The monolayer tablet according to any one of claims 1 to 10 for use in treating essential hypertension or/and reducing the risk of development of a condition associated with essential hypertension, wherein the monolayer tablet comprises:
(α) telmisartan at a dose of 40 mg and amlodipine at a dose of 5 mg;
(β) telmisartan at a dose of 40 mg and amlodipine at a dose of 10 mg;
(γ) telmisartan at a dose of 80 mg and amlodipine at a dose of 5 mg;
(δ) telmisartan at a dose of 80 mg and amlodipine at a dose of 10 mg;
(ε) telmisartan at a dose of 40 mg and hydrochlorothiazide at a dose of 12.5 mg;
(ζ) telmisartan at a dose of 40 mg and hydrochlorothiazide at a dose of 25 mg;
(η) telmisartan at a dose of 80 mg and hydrochlorothiazide at a dose of 12.5 mg;
(θ) telmisartan at a dose of 80 mg and hydrochlorothiazide at a dose of 25 mg;
(ι) telmisartan at a dose of 40 mg and indapamide at a dose of 2.5 mg;
(κ) telmisartan at a dose of 80 mg and indapamide at a dose of 2.5 mg;
(λ) telmisartan at a dose of 40 mg, amlodipine at a dose of 5 mg and hydrochlorothiazide at a dose of 12.5 mg;
(µ) telmisartan at a dose of 40 mg, amlodipine at a dose of 5 mg and hydrochlorothiazide at a dose of 25 mg;
(ν) telmisartan at a dose of 40 mg, amlodipine at a dose of 10 mg and hydrochlorothiazide at a dose of 12.5 mg;
(ξ) telmisartan at a dose of 40 mg, amlodipine at a dose of 10 mg and hydrochlorothiazide at a dose of 25 mg;
(ο) telmisartan at a dose of 80 mg, amlodipine at a dose of 5 mg and hydrochlorothiazide at a dose of 12.5 mg;
(π) telmisartan at a dose of 80 mg, amlodipine at a dose of 5 mg and hydrochlorothiazide at a dose of 25 mg;
(ρ) telmisartan at a dose of 80 mg, amlodipine at a dose of 10 mg and hydrochlorothiazide at a dose of 12.5 mg;
(ς) telmisartan at a dose of 80 mg, amlodipine at a dose of 10 mg and hydrochlorothiazide at a dose of 25 mg;
